# FASCICULE DE BREVET EUROPEEN

(11) **EP 1 313 524 B1**
(45) Date de publication et mention de la délivrance du brevet: **10.08.2005**
(21) Numéro de dépôt: 01965364.1
(22) Date de dépôt: 28.08.2001
(51) Int. Cl.: A61M 15/00, B65D 83/14

(54) **DISPOSITIF DE DISTRIBUTION DE PRODUIT FLUIDE AMELIORE**
SPENDER FÜR FLIESSFÄHIGE MEDIEN
IMPROVED FLUID PRODUCT DISPENSER

(30) Priorité: 29.08.2000 FR 0011009
(43) Date de publication de la demande: 28.05.2003
(73) Titulaire: VALOIS S.A.S., 27110 Le Neubourg (FR)
(72) Inventeur: BRUNA, Pascal, F-76300 Sotteville les Rouen (FR)
(74) Mandataire: CAPRI
(86) Numéro de dépôt international: PCT/FR2001/002682
(87) Numéro de publication internationale: WO 2002/017999

(56) Documents cités:
- WO-A-00/78378
- US-A- 3 157 317
- US-A- 3 662 958
- US-A- 5 387 034
- US-A- 5 904 139

## Description

La présente invention concerne un dispositif de distribution de produit fluide, et plus particulièrement un inhalateur de produit fluide amélioré.

Dans certains types de dispositif de distribution de produit fluide, et notamment les dispositifs du type aérosol, comportant une valve doseuse adaptée à distribuer un produit contenu dans un réservoir au moyen d'un gaz propulseur, l'unité formée par la valve doseuse et le réservoir est généralement assemblé à l'intérieur d'un corps qui incorpore un embout buccal ou nasal, et qui facilite la manipulation du dispositif. Dans certains cas, ledit corps peut en outre comporter un dispositif de déclenchement par inhalation qui coordonne l'actionnement de la valve doseuse avec une inhalation du patient. Dans ce type de dispositif, il se pose le problème de la compensation de tolérance de fabrication, aussi bien au niveau du corps, du réservoir, ou de la valve doseuse, pour éviter un assemblage de mauvaise qualité qui pourrait empêcher un fonctionnement fiable de la valve. Plus particulièrement, dans un dispositif comportant un système d'actionnement par inhalation, l'assemblage de l'unité formée du réservoir et de la valve doseuse dans le corps doit être réalisé de manière très précise pour permettre au système d'actionnement par inhalation de coopérer de manière fiable avec ladite valve doseuse. Un moyen de compenser les tolérances de fabrication est de jouer sur la course de la soupape de la valve, mais ceci n'est pas acceptable lorsque les doses à distribuer doivent être très précisément reproductibles. D'un autre coté, un assemblage dans lequel un certain jeu existe entre les différents composants, peux également empêcher un fonctionnement sûre et fiable du dispositif, ce qui n'est pas acceptable.

Le document US-5 904 139 divulgue un emboîtement à frottement cône sur cône entre la partie inférieure du corps comportant l'embout buccal et la partie supérieure du corps contenant le réservoir. Cet enfoncement peut varier légèrement pour compenser les tolérances de fabrication, un enfoncement plus important induisant des forces radiales entre les deux parties de corps.

Le document US-3 157 317 divulgue un élément déformable en élastomère disposé entre le fond du réservoir et le corps de l'inhalateur pour compenser les tolérances de fabrication. Cet élément s'il est déformé, exerce une force axiale sur le réservoir et le corps.

La présente invention a pour but de fournir un dispositif de distribution de produit fluide qui ne reproduit pas les inconvénients susmentionnés.

La présente invention a donc pour but de fournir un dispositif de distribution de produit fluide qui assure un assemblage précis de l'organe de distribution du réservoir et du corps, indépendamment des tolérances de fabrication de ces trois éléments, et sans induire des forces de compression ou de déformation entre ces éléments.

La présente invention a aussi pour but de fournir un dispositif de distribution de produit fluide qui soit simple et peu coûteux à fabriquer et à assembler.

La présente invention a également pour but de fournir un dispositif de distribution de produit fluide actionné par l'inhalation, qui permet de désolidariser l'organe de distribution du système d'actionnement par inhalation, pour permettre un actionnement manuel dudit organe de distribution.

La présente invention a donc pour objet un dispositif de distribution de produit fluide comportant un réservoir de produit, un organe de distribution, tel qu'une valve doseuse montée sur ledit réservoir, lesdits réservoir et valve étant assemblés dans un corps incorporant un orifice de distribution, caractérisé en ce que le dispositif comporte un élément de réglage disposé entre le réservoir et le corps, ledit élément de réglage comportant des moyens d'adaptations déplaçables et/ou déformables qui coopèrent avec le corps et/ou le réservoir lors de l'assemblage du réservoir dans le corps, compensant ainsi les tolérances de fabrication dudit corps et/ou dudit réservoir et/ou dudit organe de distribution.

Avantageusement, ledit élément de réglage, après assemblage du dispositif de distribution de produit fluide, n'exerce sensiblement aucune force sur le réservoir, le corps et/ou l'organe de distribution.

Avantageusement, ledit élément de réglage comporte des ouvertures axiales et les moyens d'adaptations sont réalisés sous la forme de petits cylindres pouvant coulisser à frottement dans lesdites ouvertures lors de l'assemblage du réservoir dans le corps.

Avantageusement, avant assemblage, lesdits petits cylindres sont fixés auxdites ouvertures par les ponts de matière déformables et/ou cassables.

De préférence, ledit élément de réglage est fixé sur le fond du réservoir et est monté sur le corps lors de l'assemblage dudit réservoir dans ledit corps.

Avantageusement, le montage de l'élément de réglage sur le corps est amovible.

Avantageusement, le montage de l'élément de réglage sur le corps est réalisé par une rotation, par exemple d'un quart de tour.

Avantageusement, le dispositif comporte un système de déclenchement par l'inhalation de l'organe de distribution, l'actionnement dudit organe de distribution étant désolidarisé dudit système de déclenchement par l'inhalation lorsque ledit élément de réglage est désassemblé dudit corps, permettant un actionnement manuel dudit organe de distribution.

D'autres caractéristiques et avantages de la présente invention apparaîtront plus clairement au cours de la description détaillée suivante, donnée à titre d'exemple non limitatif au regard des dessins joints, sur lesquels :
- la figure 1 est une vue schématique en perspective du dispositif selon un mode de réalisation avantageux de l'invention, avant assemblage du réservoir dans le corps ;
- la figure 2 est une vue similaire à celle de la figure 1, après assemblage du réservoir dans le corps ;
- la figure 3 est une vue schématique en section transversale dudit dispositif assemblé selon un mode de réalisation avantageux de la présente invention ;
- la figure 4 est une vue schématique partiellement découpée représentant un dispositif selon un mode de réalisation avantageux de la présente invention ;
- les figures 5 et 6 sont des vues de détails schématiques des moyens d'adaptation selon un mode de réalisation avantageux de la présente invention, respectivement avant et après assemblage du réservoir dans le corps ; et
- la figure 7 est une vue schématique de l'élément de réglage selon un mode de réalisation avantageux de la présente invention.

La présente invention va être décrite ci-après en référence à un exemple de réalisation dans lequel le dispositif de distribution de produit fluide est un inhalateur comportant une valve doseuse, et fonctionnant généralement en position inversée. L'invention ne se limite toutefois pas à ce type de dispositif, mais est au contraire applicable à tous les dispositifs de distribution de produit fluide dans lesquels peuvent se poser, lors de l'assemblage, des problèmes de tolérance de fabrication au niveau du corps, du réservoir, ou de l'élément de distribution.

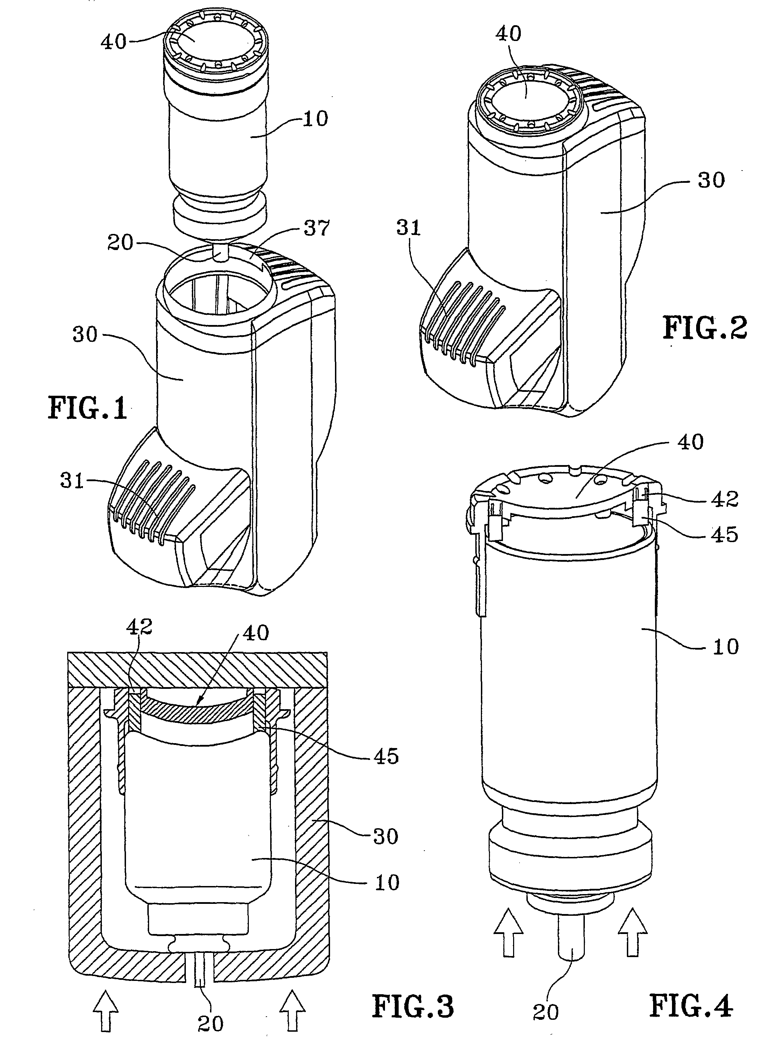

En référence aux figures 1 et 2, le dispositif de distribution comporte un réservoir 10 sur lequel est fixé un organe de distribution 20, en l'occurrence une valve doseuse, au moyen d'une bague de fixation, en l'occurrence une capsule sertie. Cette unité formée du réservoir 10 et de la valve doseuse 20 est ensuite assemblée à l'intérieur d'un corps 30 incorporant un orifice de distribution 31, qui dans l'exemple représenté est réalisé sous la forme d'un embout buccal. Il est à noter que dans l'exemple représenté sur les figures 1 et 2, cet embout buccal est recouvert d'un couvercle, celui-ci n'ayant toutefois aucune incidence sur la présente invention.

Selon l'invention, le dispositif comporte un élément de réglage 40 qui est disposé entre le réservoir 10 et le corps 30. Cet élément de réglage 40 peut par exemple être réalisé sous la forme d'une bague qui se fixe sur le fond du réservoir 10, comme visible plus clairement sur les figures 3, 4 et 7. La fixation de l'élément de réglage 40 sur le réservoir 10 peut être réalisé d'une manière quelconque, par exemple au moyen d'un encliquetage ou similaire.

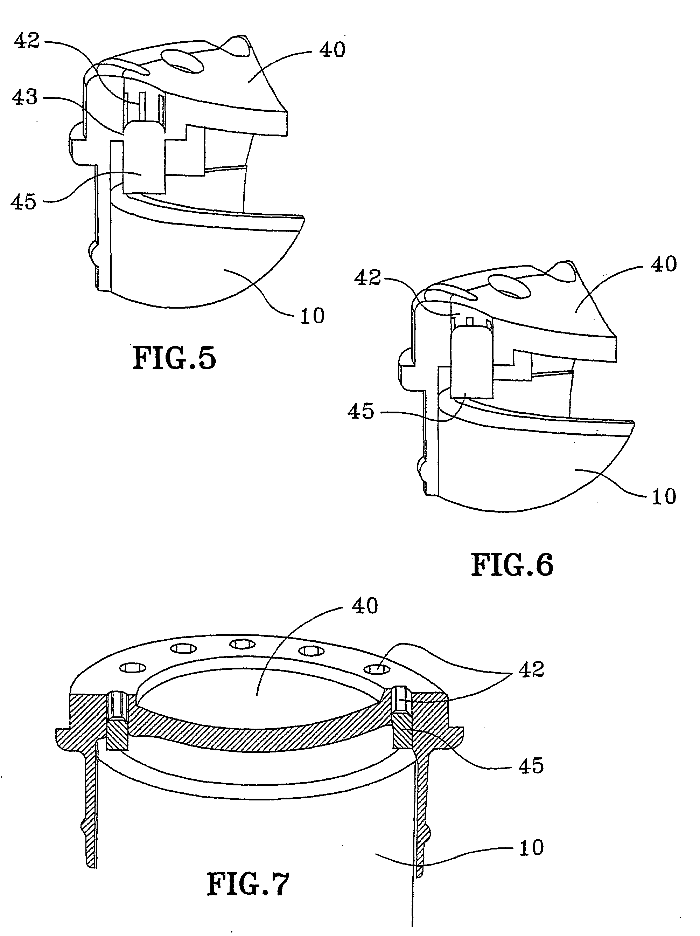

Cet élément de réglage 40 comporte des moyens d'adaptation 45 qui sont déplaçables et/ou déformables et qui coopèrent avec le corps 30 et/ou avec le réservoir 10 lors de l'assemblage dudit réservoir 10 dans ledit corps 30. Ceci permet donc, lors de l'assemblage du dispositif, de compenser toute tolérance de fabrication au niveau dudit corps, dudit réservoir et/ou dudit organe de distribution, notamment de sa soupape. Comme montré plus clairement sur les figures 3, 4, 5,6 et 7, ces moyens d'adaptation peuvent être réalisés sous la forme de petits cylindres 45 qui peuvent coulisser avec frottement à l'intérieur d'ouvertures respectives 42 disposées axialement à l'intérieur de l'élément de réglage 40. En se référant à la figure 5, qui montre le réservoir 10 avant son assemblage final dans le corps 30, l'élément de réglage 40 est pré-positionné, en l'occurrence fixé, sur le réservoir 10, et les petits cylindres 45 ne sont pas sollicités par ledit réservoir à l'intérieur desdites ouvertures respectives 42. Lorsque l'unité formée par le corps 10 et la valve doseuse 20 est assemblée à l'intérieur du corps 30, ce qui est représenté plus clairement sur les figures 3 et 4 une force axiale dans la direction des flèches sur les figures 3 et 4 est exercée par le fond du réservoir 10 sur lesdits petits cylindres 45. Ceux-ci coulissent alors avec frottement dans lesdites ouvertures axiales 42 jusqu'à ce que l'assemblage est terminé, éliminant ainsi tout jeu éventuel entre le corps 30 et la valve doseuse, entre le réservoir 10 et l'élément de réglage 40, et entre l'élément de réglage 40 et le corps 30. Avantageusement, comme représenté sur la figure 3, les petits cylindres 45 peuvent être, avant assemblage, fixés auxdites ouvertures 42 par des ponts de matières 43 qui sont déformables' et/ou cassables.

Avantageusement comme représenté sur la figure 1, l'élément de réglage 40 est fixé sur le fond du réservoir 10 et est ensuite monté sur le corps 30 lors de l'assemblage dudit réservoir 10 dans le corps 30. Plus précisément ce montage peut être réalisé de manière amovible par exemple par une rotation d'un quart de tour, ce qui est schématiquement représenté sur la figure 1 par la rainure 37 prévue à cet effet à l'intérieur de l'ouverture du corps 30 qui coopère avec ledit élément de réglage 40. Cette mise en oeuvre présente un avantage notamment dans un dispositif d'inhalation déclenché par l'inhalation de l'utilisateur. En effet, dans ce cas, le dispositif, appelé généralement MDI (Metered Dose Inhaler) comporte un système d'actionnement de la valve doseuse par l'inhalation du patient, ce système étant couplé à ladite valve doseuse. Il peut toutefois arriver qu'un actionnement manuel de la valve doseuse puisse être souhaitable dans certains cas particuliers. Ceci n'est généralement pas possible ou alors très compliqué sans un démontage complet de l'appareil. Avec la présente invention, la valve doseuse 20 peut être très simplement désolidarisée du système de déclenchement par inhalation par simple désassemblage de l'élément de réglage 40 par rapport audit corps 30. En effet, dans ce cas, le fond du réservoir devient accessible à l'utilisateur qui peut alors actionner manuellement la valve doseuse en exercent une pression axiale sur celui-ci. Le système de déclenchement par inhalation n'est pas représenté ici sur les dessins, mais il est bien connu pour l'homme du métier que ces types de systèmes, qui sont largement connus et décrits dans l'état de la technique, coopèrent avec la soupape de la valve doseuse 20. En démontant l'élément de réglage 40 par rapport au corps 30 on peut libérer provisoirement ladite soupape du système de déclenchement par inhalation, et donc actionner manuellement ladite valve doseuse.

Bien entendu l'invention n'a été décrite dans cette description qu'à l'aide de dessins très schématiques, et il est clair que diverses modifications peuvent y être apportées sans sortir du cadre de la présente invention définit par les revendications ci-jointes. En particulier, les moyens d'adaptation ne sont pas nécessairement réalisés sous la forme de petits cylindres et peuvent prendre toute forme appropriée permettant une compensation axiale des tolérances de fabrication des divers éléments à assembler dans un dispositif de distribution de produit fluide.

## Revendications

1. Dispositif de distribution de produit fluide comportant un réservoir de produit (10) et une valve doseuse (20) montée sur ledit réservoir (10), lesdits réservoir (10) et valve (20) étant assemblés dans un corps (30) incorporant un orifice de distribution (31), le dispositif comportant un élément de réglage (40) disposé entre le réservoir (10) et le corps (30), ledit élément de réglage (40) comportant des moyens d'adaptation (45) déplaçables et/ou déformables qui coopèrent avec le corps (30) et/ou le réservoir (10) lors de l'assemblage du réservoir (10) dans le corps (30), compensant ainsi les tolérances de fabrication dudit corps (30) et/ou dudit réservoir et/ou de ladite valve (20), **caractérisé en ce que** ledit élément de réglage, après assemblage du dispositif de distribution de produit fluide, n'exerçe sensiblement aucune force sur le réservoir (10), le corps (30) et/ou la valve (20).

2. Dispositif selon la revendication 1, dans lequel ledit élément de réglage (40) comporte des ouvertures axiales (42) et les moyens d'adaptation sont réalisés sous la forme de petits cylindres (45) pouvant coulisser à frottement dans lesdites ouvertures (42) lors de l'assemblage du réservoir (10) dans le corps (30).

3. Dispositif selon la revendication 2, dans lequel, avant assemblage, lesdits petits cylindres (45) sont fixés auxdites ouvertures (42) par les ponts de matière (43) déformables et/ou cassables.

4. Dispositif selon l'une quelconque des revendications précédentes, dans lequel, ledit élément de réglage (40) est fixé sur le fond du réservoir (10) et est monté sur le corps (30) lors de l'assemblage dudit réservoir (10) dans ledit corps (30).

5. Dispositif selon la revendication 4, dans lequel, le montage de l'élément de réglage (40) sur le corps (30) est amovible.

6. Dispositif selon la revendication 5, dans lequel, le montage de l'élément de réglage (40) sur le corps (30) est réalisé par une rotation par exemple d'un quart de tour.

7. Dispositif selon la revendication 5 ou la revendication 6, dans lequel, le dispositif comporte un système de déclenchement par l'inhalation de la valve (20), l'actionnement de ladite valve (20) étant désolidarisé dudit système de déclenchement par l'inhalation lorsque ledit élément de réglage (40) est désassemblé dudit corps (30), permettant un actionnement manuel de ladite valve (20).

## Patentansprüche

1. Einrichtung zum Verteilen eines Fluidproduktes, umfassend einen Produktbehälter (10) und ein Dosierventil (20), welches an dem Behälter (10) befestigt ist, wobei Behälter (10) und Ventil (20) in einem Gehäuse (30) montiert sind, welches eine Verteileröffnung (31) aufweist, wobei die Einrichtung ein Regelelement (40) umfasst, welches zwischen dem Behälter (10) und dem Gehäuse (30) angeordnet ist, wobei das Regelelement (40) verstellbare und/oder verformbare Adaptionsmittel (45) umfasst, welche bei der Montage des Behälters (10) in dem Gehäuse (30) mit dem Gehäuse (30) und/oder dem Behälter (10) zusammenwirken, um so die Herstellungstoleranzen des Gehäuses (30) und/oder des Behälters und/oder des Ventils (20) zu kompensieren, **dadurch gekennzeichnet, dass** das Regelelement nach der Montage der Einrichtung zum Verteilen eines Fluidproduktes im wesentlichen keine Kraft auf den Behälter (10), das Gehäuse (30) und/oder das Ventil (20) ausübt.

2. Einrichtung nach Anspruch 1, bei welcher das Regelelement (40) axiale Öffnungen (42) aufweist und die Adaptionsmittel in der Form von kleinen Zylindern (45) ausgebildet sind, welche während der Montage des Behälters (10) im Gehäuse (30) mit Reibung in den Öffnungen (42) gleiten können.

3. Einrichtung nach Anspruch 2, bei welcher vor der Montage die kleinen Zylinder (45) mit den Öffnungen (42) über die verformbaren und/oder abreißbaren Materialbrücken (43) verbunden sind.

4. Einrichtung nach einem der vorangehenden Ansprüche, bei welcher das Regelelement (40) auf dem Boden des Behälters (10) fixiert ist und an dem Gehäuse (30) bei der Montage dieses Behälters (10) im Gehäuse (30) montiert wird.

5. Einrichtung nach Anspruch 4, bei welcher die Befestigung des Regelelementes (40) an dem Gehäuse (30) lösbar ist.

6. Einrichtung nach Anspruch 5, bei welcher die Befestigung des Regelelementes (40) an dem Gehäuse (30) durch eine Drehung, beispielsweise eine Viertelumdrehung bewerkstelligt wird.

7. Einrichtung nach Anspruch 5 oder Anspruch 6, bei welcher die Einrichtung ein System zum Auslösen des Ventils (20) durch den Inhaliervorgang umfasst, wobei die Betätigung dieses Ventils (20) von dem System zum Auslösen durch den Inhaliervorgang getrennt wird, wenn das Regelelement (40) von dem Gehäuse (30) demontiert wird, wodurch eine manuelle Betätigung dieses Ventils (20) ermöglicht wird.

## Claims

1. A fluid dispenser device comprising a fluid reservoir (10), and a metering valve (20), fitted to said reservoir (10), said reservoir (10) and said valve (20) being assembled into a body (30) incorporating a dispensing orifice (31), said device including an adjustment element (40) disposed between the reservoir (10) and the body (30), said adjustment element (40) being provided with adapter means (45) that are movable and/or deformable and that co-operate with the body (30) and/or with the reservoir (10) while the reservoir (10) is being assembled into the body (30), thereby compensating for the manufacturing tolerances of said body (30) and/or of said reservoir and/or said valve (20), **characterized in that** said adjustment element exerts substantially no force on the reservoir (10), on the body (30), and/or on the valve (20), once the fluid dispenser device has been assembled.

2. A device according to claim 1, in which the adjustment element (40) is provided with axial openings (42), and the adapter means are implemented in the form of small cylinders (45) that can slide snugly in said openings (42) while said reservoir (10) is being assembled into the body (30).

3. A device according to claim 2, in which, prior to assembly, said small cylinders (45) are fixed to said openings (42) by bridges of material (43) that are deformable and/or breakable.

4. A device according to any preceding claim, in which said adjustment element (40) is fixed to the bottom of the reservoir (10) and is fitted to the body (30) while said reservoir (10) is being assembled into said body (30).

5. A device according to claim 4, in which the adjustment element (40) is fitted to the body (30) removably.

6. A device according to claim 5, in which the adjustment element (40) is fitted to the body (30) by turning e.g. through one fourth of a turn.

7. A device according to claim 5 or claim 6, in which the device includes an inhalation-driven trigger system for triggering the valve (20), actuation of said valve (20) being separated from said inhalation-driven trigger system when said adjustment element (40) is disassembled from said body (30), thereby enabling said valve (20) to be actuated manually.
